# EUROPEAN PATENT SPECIFICATION

(11) **EP 3 787 567 B1**
(45) Date of publication and mention of the grant of the patent: **09.12.2026**
(21) Application number: 19723274.7
(22) Date of filing: 29.04.2019
(51) Int. Cl.: A61F 5/451, A61F 5/455

(54) **FLUID COLLECTION SYSTEMS**
FLÜSSIGKEITSSAMMELSYSTEME
SYSTÈMES DE COLLECTE DE FLUIDE

(30) Priority: 01.05.2018 US 201862665302 P
(43) Date of publication of application: 10.03.2021
(60) Divisional application: 26215830.6
(73) Proprietor: Purewick Corporation, El Cajon, California 92020 (US)
(72) Inventor: GLITHERO, Jason Iain, McDonough, Georgia 30252 (US)
(74) Representative: Hoffmann Eitle
(86) International application number: PCT/US2019/029608
(87) International publication number: WO 2019/212949

(56) References cited:
- EP-A1- 1 382 318
- US-A- 5 002 541
- US-A1- 2002 087 131
- US-A1- 2018 028 349

## Description

### TECHNICAL FIELD

The present disclosure relates to a portable fluid collection system to be worn by a female user while working as a pilot or driver or in a hazardous area.

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, can be used to address some of these circumstances. However, bed pans and urinary catheters have several problems associated therewith. For example, bed pans can be prone to discomfort, spills, and other hygiene issues. Urinary catheters can be uncomfortable, painful, and can cause urinary tract infections.

US 2018/0028349 A1 discloses a system for collecting and transporting urine away from the body of a person via a discharge line that delivers urine to an external urine receptacle that has a partial vacuum imposed on it by a vacuum source connected to the receptacle by a vacuum line.

The disclosure of US 2002/0087131 A is similar, but includes a urine sensor arranged to open a valve in the urine discharge line to allow the partial vacuum in the external urine collection receptacle to impose itself on the interface adjacent the urethra of the user of the device.

In these respects, the disclosure of US 5,002,541 is similar. The external collection receptacle is preferably large enough to store all the urine which would normally be excreted over a 24 hours period, and can be supported within a housing that is free-standing and can be served by an external supply of A.C. electricity at mains (e.g. 110v) voltage.

EP-A1-1382318 discloses a urine collection and storage device which features a body that contains a small urine storage container, pump, motor and battery. It is connected by a hinge to a bucket that can be offered up to the urethra of a user, with the pump drawing urine from the bucket into the storage container, whereupon the bucket can be again folded flat against the body. The device is small and light enough to carry about the person, ready for intermittent active use during the next-occurring urination event.

Thus, users and manufacturers of fluid collection devices continue to seek new and improved devices, systems, and methods to collect urine.

### SUMMARY

The invention is defined in the independent claim below. The dependent claims are directed to optional features and preferred embodiments. Disclosed herein are devices, systems, and methods of using fluid collection devices..

A method to collect fluid is described herein. The method includes positioning an opening of a fluid collection device adjacent to a female urethra, the opening defined by a fluid impermeable barrier of the fluid collection device. The method includes receiving fluid from the female urethra into a chamber of the fluid collection device, the chamber of the fluid collection device at least partially defined by the fluid impermeable barrier. The method includes applying suction with a portable vacuum source effective to suction the fluid from the chamber via a conduit disposed therein and in fluid communication with the portable suction device.

[deleted]

Features from any of the disclosed embodiments may be used in combination with one another. In addition, other features and advantages of the present invention will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
FIG. 1 is a block diagram of a system for fluid collection.
FIGS. 2-4 are schematic cross-sectional views of female fluid collection devices, according to embodiments.
FIGS. 5-7 have been excised.
FIG. 8 is a flow diagram of a method to collect fluid.

### DETAILED DESCRIPTION

The fluid collection devices and systems described below include a portable vacuum source to remove urine from the fluid collection device. The portable vacuum source allows portable usage of the systems such as in non-hospital environments.

In an example, a fluid collection device includes a fluid impermeable barrier that at least partially defines a chamber. The fluid impermeable barrier also defines an opening extending therethrough that is configured to be positioned adjacent to a female urethra or have a male urethra positioned therethrough. The fluid collection device also includes a tube having a channel extending between an inlet and outlet thereof. The inlet is configured to be coupled to a suction source and the outlet is configured to be in fluid communication with (e.g., fluidly coupled to) a fluid storage (vessel or container). The outlet is positioned downstream from the inlet. The channel also defines at least one aperture therein that fluidly couples an interior of the channel to the rest of the chamber.

The fluid collection devices disclosed herein are configured to collect fluid(s) from an individual. The fluid collected by the fluid collection devices can include urine.

The fluid collection devices disclosed herein are configured to be used in fluid collection systems. Fluid collection systems may include a gas source. Systems that include a gas source can, in some examples, resolve several problems associated with systems that include a vacuum source. For example, a system that includes a vacuum source draws fluid(s) towards the vacuum source and deposits most of the fluid(s) in a fluid storage container before the fluid(s) can reach the vacuum source. However, a small quantity of fluid(s) (e.g., vapor from the fluid(s)) can still reach the vacuum source, which can contaminate and/or damage (e.g., rust) the vacuum source. Additionally, a large quantity of the fluid(s) can reach the vacuum source when the fluid storage container is substantially full. However, a system that includes a gas source (e.g., compressed air) moves the fluid(s) away from the gas source, thereby preventing contamination and/or damage though splashing or spray of the fluid(s) may be a problem with a gas. For example, a gas source may be used to create a vacuum by flowing a gas past a connected end of the conduit at a perpendicular or oblique angle to the conduit to create a vacuum in the conduit. The fluids are pulled up the conduit and into the gas flow in the direction of the gas flow, which is away from the gas source. In another example, systems that include a vacuum source cannot be used in environments that do not include an available vacuum source (e.g., a patient's room does not include a vacuum source or the vacuum source is being used). As such, systems that include a gas source can be used in environments that do not include an available vacuum source. A liquid source such as water can be used to create and implement a vacuum in the same way as the gas source. The vacuum source can be utilized with any of the devices or systems disclosed herein to remove a fluid therefrom.

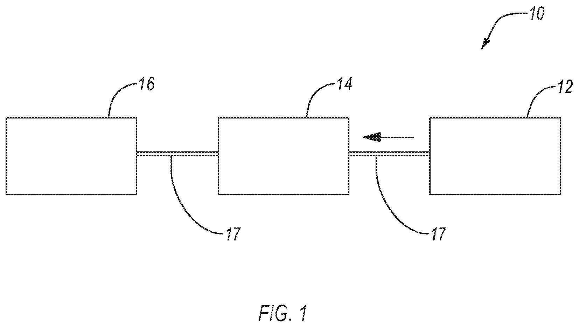

FIG. 1 is a block diagram of a system 10 for fluid collection. The system 10 includes a fluid collection device 12, a fluid storage container 14, and a portable vacuum source 16. The fluid collection device 12, the fluid storage container 14, and the portable vacuum source 16 may be fluidly coupled to (e.g., in fluid communication with) each other via one or more conduits 17. For example, fluid collection device 12 may be in fluid communication with one or more of the fluid storage container 14 or the portable vacuum source via the conduit 17. Fluid (e.g., urine or other bodily fluids) collected in the fluid collection device 12 may be removed from the fluid collection device 12 via the conduit 17 which protrudes into an interior region of the fluid collection device 12. For example, a first open end of the conduit 17 may extend into the fluid collection device 12 to a reservoir therein. The second open end of the conduit 17 may extend into the fluid storage container 14 or the portable vacuum source 16. The suction force may be introduced into the interior region of the fluid collection device 12 via the first open end of the conduit 17 responsive to a suction (e.g., vacuum) force applied at the second end of the conduit 17. The suction force may be applied to the second open end of the conduit 17 by the portable vacuum source 16 either directly or indirectly.

The suction force may be applied indirectly via the fluid storage container 14. For example, the second open end of the conduit 17 may be disposed within the fluid storage container 14 and an additional conduit 17 may extend from the fluid storage container 14 to the portable vacuum source 16. Accordingly, the portable vacuum source 16 may apply suction to the fluid collection device 12 via the fluid storage container 14. The suction force may be applied directly via the fluid storage container 14. For example, the second open end of the conduit 17 may be disposed within the portable vacuum source 16. An additional conduit 17 may extend from the portable vacuum source 16 to a point outside of the fluid collection device 12, such as to the fluid storage container. In such examples, the portable vacuum source 16 may be disposed between the fluid collection device 12 and the fluid storage container 14.

The fluid collection device 12 is shaped and sized to be positioned adjacent to a female urethra. The fluid collection device 12 includes a fluid impermeable barrier at least partially defining a chamber (e.g., interior region of the fluid collection device 12) of the fluid collection device. The fluid impermeable barrier also defines an opening extending therethrough from the external environment. The opening may be positioned adjacent to a female urethra. The fluid collection device 12 may include a fluid permeable membrane disposed within the fluid impermeable barrier. The fluid collection device 12 may include a fluid permeable support disposed within the fluid permeable membrane. The conduit 17 extends into the fluid collection device 12 at a first end region, through one or more of the fluid impermeable barrier, fluid permeable membrane, or the fluid permeable support to a second end region of the fluid collection device 12. Exemplary fluid collection devices for use with the systems and methods herein are described in more detail below.

In some examples, the fluid storage container 14 may include a bag (e.g., drainage bag), a bottle or cup (e.g., collection jar), or any other enclosed container for storing bodily fluid(s) such as urine. In some examples, the conduit 17 may extend from the fluid collection device and attach to the fluid storage container 14 at a first point therein.

The portable vacuum source 16 is disposed in or on the fluid collection device 12. Fluid, such as urine, is drained from the fluid collection device 12 using the portable vacuum source 16. The portable vacuum source 16 may include one or more of a manual vacuum pump, and electric vacuum pump, a diaphragm pump, a centrifugal pump, a displacement pump, a magnetically driven pump, a peristaltic pump, or any pump configured to produce a vacuum. The portable vacuum source 16 provides a vacuum or suction to remove fluid from the fluid collection device 12. In some examples, the portable vacuum source 16 may be powered by one or more of a power cord (e.g., connected to a power socket), one or more batteries, or even manual power (e.g., a hand operated vacuum pump). The portable vacuum source 16 is sized and shaped to fit outside of, on, or within the fluid collection device 12. For example, the portable vacuum source 16 may include one or more miniaturized pumps or one or more micro pumps. The portable vacuum sources 16 disclosed herein may include one or more of a switch, a button, a plug, a remote, or any other device suitable to activate the portable vacuum source. It should be understood that the portable vacuum sources 16 disclosed herein provides a portable means of providing a suction or vacuum that allows use of the devices and systems herein outside of hospital or care facility environments where vacuum lines are plumbed into patient rooms or large (e.g., larger or heavier than a patient can readily carry) vacuum sources are located. In particular, the portable vacuum source is small and light enough to be carried by a user (e.g., patient) during transportation of the user.

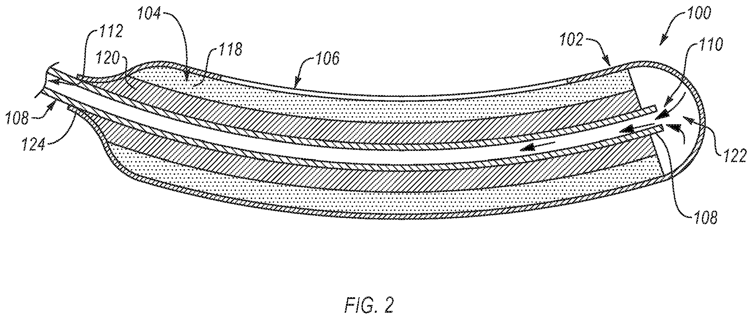

FIG. 2 is a schematic cross-sectional view of a fluid collection device 100. The fluid collection device 100 is an example of a female fluid collection device 100 that is configured to receive fluid(s) from a female. The fluid collection device 100 includes a fluid impermeable barrier 102. The fluid impermeable barrier 102 at least partially defines a chamber 104 (e.g., interior region) and an opening 106. The opening 106 is formed in and extends through the fluid impermeable barrier 102, thereby enabling fluid(s) to enter the chamber 104 from outside of the fluid collection device 100. The opening 106 is configured to be positioned adjacent to a female urethra. The fluid collection device 100 also includes conduit 108 that is at least partially disposed in the chamber 104. The conduit 108 (e.g., a tube) includes an inlet 110 at a first end region and an outlet 112 at a second end region positioned downstream from the inlet 110. The conduit 108 fluidly couples an interior region of the chamber 104 with the fluid storage container (not shown) or the portable vacuum source (not shown).

In the illustrated embodiment, the conduit 108 is at least partially disposed in the chamber 104. The conduit 108 extends into the fluid impermeable barrier 102 from the first end region (e.g., proximate to the outlet 112) and extends to the second end region (e.g., opposite the first end region) to a point proximate to a reservoir 122 such that the inlet 110 is in fluid communication with the reservoir 122. For example, in the illustrated embodiment, the inlet 110 is positioned in the reservoir 122. However, in other examples, the inlet 110 may be positioned flush with or behind an end of the fluid permeable membrane 118 that partially defines the reservoir 122. The fluid collected in the fluid collection device 100 may be removed from the interior region of the chamber 104 via the conduit 108. The conduit 108 may include a flexible material such as plastic tubing (e.g., medical tubing). Such plastic tubing may include a thermoplastic elastomer, polyvinyl chloride, ethylene vinyl acetate, polytetrafluoroethylene, etc., tubing. In some examples, the conduit 108 may include silicon or latex. In some examples, the conduit 108 may include one or more portions that are resilient, such as to by having one or more of a diameter or wall thickness that allows the conduit to be flexible.

The fluid collection device 100 may be positioned proximate to the female urethra and urine may enter the interior region of the fluid collection device 100 via the opening 106. The fluid collection device 100 is configured to receive the fluid(s) into the chamber 104 via the opening 106. The opening 106 exhibits an elongated shape that is configured to extend from a first location below the urethral opening near the anus to a second location above the urethral opening at or near the clitoris. The opening 106 exhibits an elongated shape since the space between the legs of a female is relatively small when the legs of the female are closed, thereby only permitting the flow of the fluid(s) along a path that corresponds to the elongated shape of the opening 106 (e.g., longitudinally extending opening). The longitudinal axis or dimension of the fluid collection devices disclosed herein refers to the axis or dimension that is parallel to largest dimension of the device, axially along a cylindrical device as show in FIG. 2. The opening 106 in the fluid impermeable barrier 102 can exhibit a width that is measured transverse to the longitudinal direction of the fluid collection device 100 and may be at least about 10% of the circumference of the fluid collection device 100, such as about 25% to about 50%, about 40% to about 60%, about 50% to about 75%, about 65% to about 85%, or about 75% to about 100% of the circumference of the fluid collection device 100. The opening 106 can exhibit a width that is greater than 50% of the circumference of the fluid collection device 100 since the vacuum (e.g., suction) through the conduit 108 pulls the fluid into the conduit 108. The opening 106 is vertically oriented having a major axis parallel to the longitudinal axis of the device 100. In an example, the fluid impermeable barrier 102 can be configured to be attached to the individual, such as adhesively attached (e.g., with a hydrogel adhesive) to the individual. According to an example, a suitable adhesive is a hydrogel layer.

The fluid impermeable barrier 102 may also temporarily store the fluid(s in the chamber 104. For example, the fluid impermeable barrier 102 can be formed of any suitable fluid impermeable materials, such as a fluid impermeable polymer (e.g., silicone, polypropylene, polyethylene, polyethylene terephthalate, a polycarbonate, etc.), a metal film, another suitable material, or combinations thereof. As such, the fluid impermeable barrier 102 substantially prevents the fluid(s) from exiting the portions of the chamber 104 that are spaced from the opening 106. The fluid impermeable barrier 102 stores fluid(s) in a reservoir 122 therein, positioned in the second end region of the chamber 104. In an example, the fluid impermeable barrier 102 can be air permeable and fluid impermeable. In such an example, the fluid impermeable barrier 102 can be formed of a hydrophobic material that defines a plurality of pores. In an example, at least one or more portions of at least an outer surface of the fluid impermeable barrier 102 can be formed from a soft and/or smooth material, thereby reducing chaffing. The fluid impermeable barrier 102 may include markings thereon, such as one or more markings to aid a user in aligning the device 100 on the wearer. For example, a line on the fluid impermeable barrier 102 (e.g., opposite the opening 106) may allow a healthcare professional to align the opening 106 over the urethra of the wearer. In examples, the markings may include one or more of alignment guide or an orientation indicator, such as a stripe or hashes. Such markings may be positioned to align the device 100 to one or more anatomical features such as a pubic bone, etc.

The fluid collection device 100 can include a fluid permeable membrane 118 disposed in the chamber 104. The fluid permeable membrane 118 can cover at least a portion (e.g., all) of the opening 106. The fluid permeable membrane 118 can be configured to wick any fluid away from the opening 106, thereby preventing the fluid from escaping the chamber 104. The permeable properties referred to herein can be wicking, capillary action, diffusion, or other similar properties or processes, and are referred to herein as "permeable" and/or "wicking." Such "wicking" may not include absorption into the wicking material. The fluid permeable membrane 118 can also wick the fluid generally towards an interior of the chamber 104, as discussed in more detail below. The fluid permeable membrane 118 can include any material that can wick the fluid. For example, the fluid permeable membrane 118 can include fabric, such as a gauze (e.g., a silk, linen, polymer based materials such as polyester, or cotton gauze), another soft fabric (e.g., jersey knit fabric or the like), or another smooth fabric (e.g., rayon, satin, or the like). Forming the fluid permeable membrane 118 from gauze, soft fabric, and/or smooth fabric can reduce chaffing caused by the fluid collection device 100.

The fluid collection device 100 can include a fluid permeable support 120 disposed in the chamber 104. The fluid permeable support 120 is configured to support the fluid permeable membrane 118 since the fluid permeable membrane 118 can be formed from a foldable, flimsy, or otherwise easily deformable material. For example, the fluid permeable support 120 can be positioned such that the fluid permeable membrane 118 is disposed between the fluid permeable support 120 and the fluid impermeable barrier 102. As such, the fluid permeable support 120 can support and maintain the position of the fluid permeable membrane 118. The fluid permeable support 120 can include any material that can wick the fluid. The fluid permeable support 120 can be formed from any fluid permeable material that is less deformable than the fluid permeable membrane 118. For example, the fluid permeable support 120 can include a porous nylon structure. In an example, the fluid permeable support 120 can be omitted from the fluid collection device 100.

In an example, the fluid permeable membrane 118 and the fluid permeable support 120 can at least substantially completely fill the portions of the chamber 104 that are not occupied by the conduit 108. In another example, the fluid permeable membrane 118 and the fluid permeable support 120 may not substantially completely fill the portions of the chamber 104 that are not occupied by the conduit 108. In such an example, the fluid collection device 100 includes the reservoir 122 disposed in the chamber 104. The reservoir 122 is a substantially unoccupied portion of the chamber 104. The reservoir may be defined between the fluid impermeable barrier 102 and one or both of the fluid permeable membrane 118 and the fluid permeable support. The fluid(s) that are in the chamber 104 can flow through the fluid permeable membrane 118 and/or fluid permeable support 120 to the reservoir 122. The reservoir 122 can store at least some of the fluid(s) therein.

The reservoir 122 is located at the portion of the chamber 104 that is closest to the inlet 110 (i.e., the second end region). However, the reservoir 122 can be located at different locations in the chamber 104.

The fluid collection device 100 is cylindrical, for complementing or contouring to the vaginal region of a female subject. The cross-sectional shape of the fluid collection devices disclosed herein may include any of various sizes.

The fluid impermeable barrier 102, the fluid permeable membrane 118 and the fluid permeable support 120 can be configured to have the conduit 108 at least partially disposed in the chamber 104. For example, at least one of the fluid permeable membrane 118 and the fluid permeable support 120 can be configured to form a space that accommodates the conduit 108. In another example, the fluid impermeable barrier 102 can define an aperture 124 sized to receive the conduit 108 (e.g., at least one tube). The at least one conduit 108 can be disposed in the chamber 104 via the aperture 124. The apertures 124 can be configured to form an at least substantially fluid tight seal against the conduit 108 or the at least one tube thereby substantially preventing the fluid(s) from escaping the chamber 104. The fluid collected in the fluid collection device 100 is removed from the interior region of the chamber 104 via the conduit 108. As shown in FIG. 2, the end of the conduit 108 extends beyond the fluid permeable membrane 118 and/or fluid permeable support 120, and into the reservoir 122.

As previously discussed, the conduit 108 is configured to be coupled to the fluid storage container.

Locating the inlet 110 at or near a gravimetrically low point of the chamber 104 enables the conduit to receive more of the fluid(s) than if inlet 110 was located elsewhere and reduce the likelihood of pooling (e.g., pooling of the fluid(s) can cause microbe growth and foul odors). For instance, the fluid(s) in the fluid permeable membrane 118 and the fluid permeable support 120 can flow in any direction due to capillary forces. However, the fluid(s) may exhibit a preference to flow in the direction of gravity, especially when at least a portion of the fluid permeable membrane 118 and/or the fluid permeable support 120 is saturated with the fluid(s). Accordingly, the inlet 110 and the reservoir 122 are located in the fluid collection device in a position expected to be the gravimetrically low point in the fluid collection device when worn by a user.

As the portable vacuum source (FIG. 1) applies a vacuum/suction in the conduit 108, the fluid(s) in the chamber 104 (e.g., at the second end region such as in the reservoir 122) may be drawn into the inlet 110 and out of the fluid collection device 100 via the conduit 108. In some examples, the conduit may be frosted or opaque (e.g., black) to obscure visibility of the fluid(s) therein.

In an example, the conduit 108 is configured to be at least insertable into the chamber 104. In such an example, the conduit 108 can include one or more markers (not shown) on an exterior thereof that are configured to facilitate insertion of the conduit 108 into the chamber 104. For example, the conduit 108 can include one or more markings thereon that are configured to prevent over or under insertion of the conduit 108, such as when the conduit 108 defines an inlet 110 that is configured to be disposed in or adjacent to the reservoir 122. In another example, the conduit 108 can include one or more markings thereon that are configured to facilitate correct rotation of the conduit 108 relative to the chamber 104. In an example, the one or more markings can include a line, a dot, a sticker, or any other suitable marking.

In an example, one or more components of the fluid collection device 100 can include an antimicrobial material, such as an antibacterial material where the fluid collection device may contact the wearer or the bodily fluid of the wearer. The antimicrobial material can include an antimicrobial coating, such as a nitrofurazone or silver coating. The antimicrobial material can inhibit microbial growth, such as microbial growth due to pooling or stagnation of the fluid(s). In an example, one or more components (e.g., impermeable barrier 102, conduit 108, etc.) of the fluid collection device 100 can include an odor blocking or absorbing material such as a cyclodextrine containing material or a thermoplastic elastomer (TPE) polymer.

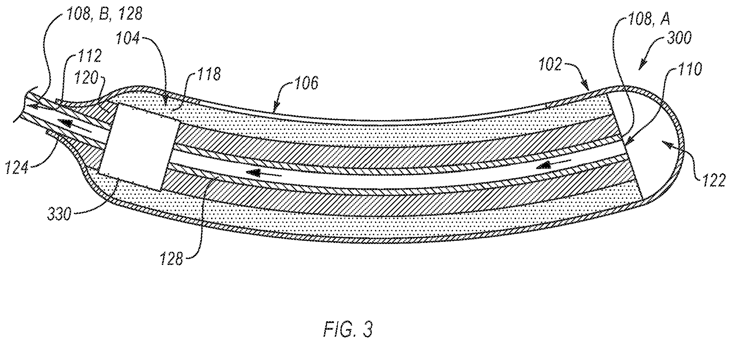

FIG. 3 is a schematic cross-sectional view of a fluid collection device 300, according to an embodiment. The fluid collection device 300 includes the portable vacuum source 330 disposed therein. Except as otherwise disclosed herein, the fluid collection device 300 can be the same as or substantially similar to the fluid collection device 100 of FIG. 2, in one or more aspects. For example, the fluid collection device 300 includes the fluid impermeable barrier 102 that defines the chamber 104 and the opening 106. The fluid collection device 300 also includes at least one of the fluid permeable membrane 118, the fluid permeable support 120, and the reservoir 122 disposed in the chamber 104.

The fluid collection device 300 includes the conduit 108 that is at least partially disposed in the chamber 104. The conduit 108 includes one or more walls that define an inlet 110 and the outlet 112. The inlet 110 enables at least some of the fluids that are present in the chamber 104 to enter the conduit 108. The conduit 108 is configured to have the inlet 110 located at, near, or spaced at, a gravimetrically low point of the chamber 104. The conduit 108 is configured to have the at least one inlet 110 disposed in or adjacent to the reservoir 122.

The conduit 108 is in fluid communication with the interior region of the chamber 104 via the fluid impermeable barrier 102. As such, the fluid impermeable barrier 102 defines an aperture 124. In an example, as illustrated, the aperture 124 enables the conduit 108 to extend outwardly from the chamber 104 when the conduit 108 is only partially disposed in the chamber 104. In some examples, the conduit 108 may include a plurality of separate sections. For example and as shown, the conduit 108 may include a first section A and a second section B. The first section A may include the inlet 110 extending from the distal end (e.g., first end region) to the portable vacuum source 330 and the B section may extend from the portable vacuum source 330 out of the aperture 124, such as to a fluid storage container (not shown).

The portable vacuum source 330 may include a pump, such as any of the portable vacuum pumps disclosed herein. For example, the portable vacuum source 330 may include a manual vacuum pump, a diaphragm pump, a centrifugal pump, a displacement pump, a magnetically driven pump, a peristaltic pump, or any pump configured to produce suction, a vacuum, or otherwise move a fluid. The portable vacuum source 330 may be sized to fit in the chamber 104 inside of the fluid impermeable barrier 102. In some examples, the portable vacuum source 330 may be sealed in a fluid tight housing or container. The portable vacuum source 330 may apply a vacuum (e.g., suction) in the A section of the conduit 108 effective to suction fluid from the chamber 104. The fluid may travel through the A section to the B section (e.g., through the portable vacuum source 330) and out of the fluid collection device 300 via the B section by flow induced by the vacuum or suction applied by the portable vacuum source 330. For example, the portable vacuum source 330 may include a centrifugal pump and an impeller therein may draw the fluid from the chamber 104 via the inlet 110 and force the fluid out of the chamber 104 via the B section of the conduit 108. Each of the A second and the B section of the conduit 108 may be fluidly coupled (e.g., sealed) to the portable vacuum source 330.

In some examples, the portable vacuum source 330 and the conduit 108 can be integrally formed together (e.g., exhibit single piece construction).

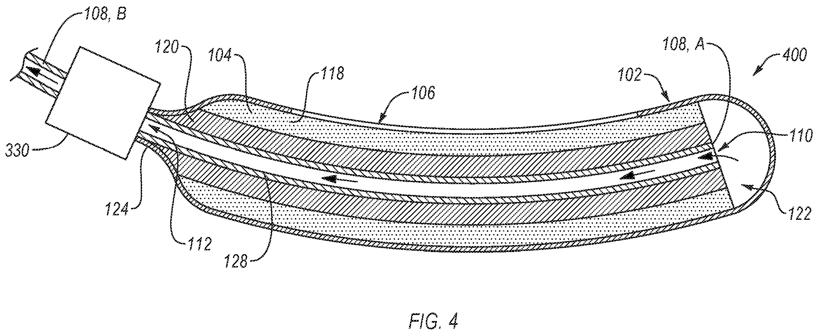

FIG. 4 is a schematic cross-sectional view of a fluid collection device 400, according to an embodiment. The fluid collection device 400 includes the portable vacuum source 330 disposed thereon. Except as otherwise disclosed herein, the fluid collection device 400 can be the same as or substantially similar to the fluid collection device 100 of FIG. 2, in one or more aspects. For example, the fluid collection device 400 includes the fluid impermeable barrier 102 that defines the chamber 104 and the opening 106. The fluid collection device 400 also includes at least one of the fluid permeable membrane 118, the fluid permeable support 120, and the reservoir 122 disposed in the chamber 104. The portable vacuum source 330 is attached to the fluid collection device 400 at the fluid impermeable barrier 102.

The fluid collection device 400 includes the conduit 108 that is at least partially disposed in the chamber 104. The conduit 108 includes one or more walls that define an inlet 110 and the outlet 112. The inlet 110 enables at least some of the fluids that are present in the chamber 104 to enter the conduit 108. The conduit 108 is configured to have the inlet 110 located at, near, or spaced at a gravimetrically low point of the chamber 104. The conduit 108 is configured to have the at least one inlet 110 disposed in or adjacent to the reservoir 122.

The conduit 108 is in fluid communication with the interior region of the chamber 104 via the fluid impermeable barrier 102. As such, the fluid impermeable barrier 102 can define an aperture 124. In an example, as illustrated, the aperture 124 enables the conduit 108 to extend outwardly from the chamber 104 when the conduit 108 is only partially disposed in the chamber 104. In some examples, the conduit 108 may include a plurality of separate sections. For example and as shown, the conduit 108 may include the first section A and the second section B. The first section A may include the inlet 110 extending from the distal end (e.g., first end region), out of the aperture 124, to the portable vacuum source 330 mounted thereto. The portable vacuum source 330 may be mounted to the outer surface of the fluid collection device 400, such as on the fluid impermeable barrier 102. The B section may be attached to and extend from the portable vacuum source 330, such as to a fluid storage container (not shown).

The portable vacuum source 330 may include any of the portable vacuum pumps disclosed herein such as a manual vacuum pump, a diaphragm pump, a centrifugal pump, a displacement pump, a magnetically driven pump, a peristaltic pump, or any pump configured to produce a vacuum. The portable vacuum source 330 may be sized to fit in the chamber 104 inside of the fluid impermeable barrier 102. In some examples, the portable vacuum source 330 may be sealed in a fluid tight housing or container. The portable vacuum source 330 applies a vacuum (e.g., suction) in the A section of the conduit 108 effective to suction fluid from the chamber 104. The fluid travels through the A section out of the fluid collection device 400 to the portable vacuum source 330. The fluid is removed from the portable vacuum source 330 via the B section by flow induced by the vacuum or suction applied by the portable vacuum source 330. For example, the portable vacuum source 330 may include a centrifugal pump and an impeller therein may draw the fluid from the chamber 104 via the inlet 110 and suction the fluid out of the chamber 104 via the portable vacuum source 330 to the B section of the conduit 108. Each of the A section and the B section of the conduit 108 is fluidly coupled (e.g., sealed) to the portable vacuum source 330. In some examples, the portable vacuum source 330 and the conduit 108 (e.g., one or both of the A section or the B section) can be integrally formed together to exhibit single piece construction. The female fluid collection devices disclosed herein may also include one or more vacuum relief holes in the fluid impermeable barrier 102 to control an amount of suction or vacuum in the chamber 104. The vacuum relief hole may be positioned at any point on the fluid permeable membrane, such as at an intermediate point between the reservoir 122 and the outlet 112. Such vacuum relief holes may allow the chamber 104 to remain substantially at atmospheric pressure to prevent the fluid collection devices from deforming under vacuum force.

In any of the examples disclosed herein, the conduits 108 may include or be operably coupled to a flow meter (not shown) to measure the flow of fluid(s) therein, one or more securement devices (e.g., a STATLOCK^{®} securement device, not shown) or fittings to secure the conduit 108 to one or more components of the systems or devices disclosed herein (e.g., portable vacuum source or fluid storage container), or one or more valves to control the flow of fluid(s) in the systems and devices herein. In any of the examples disclosed herein, the conduits 108 or other portions of the devices and systems may include a port for inserting one or more testing devices to determine if the collected urine indicates an onset of an infection (e.g., urinary tract infection or kidney infection). For example, one or more testing strips or sticks may be inserted through a port in the conduit 108.

In an example, at least one of portion of the conduit 108 of the fluid collection devices or systems herein can be formed of an at least partially opaque material which can obscure the fluids that are present therein. For example, the B section of the conduits 108 disclosed herein may be formed of an opaque material or translucent material while the A section may be formed of a transparent material or translucent material. In some examples, the B section may include transparent or translucent material. Unlike the opaque or nearly opaque material, the translucent material allows a user of the devices and systems herein to visually identify fluid(s) or issues that are inhibiting the flow of fluid(s) within the conduit 108.

In any of the example, systems or devices disclosed herein, the system of fluid collection device may include moisture sensors (not shown) disposed inside of the chamber of the fluid collection device. In such examples, the moisture sensor may be operably coupled to a controller or directly to the portable vacuum source, and may provide electrical signals indicating that moisture is or is not detected in one or more portions of the chamber. The moisture sensor(s) may provide an indication that moisture is present, and responsive thereto, the controller or portable vacuum device may direct the initiation of suction to the chamber to remove the fluid therefrom. Suitable moisture sensors may include capacitance sensors, volumetric sensors, potential sensors, resistance sensors, frequency domain reflectometry sensors, time domain reflectometry sensors, or any other suitable moisture sensor. In practice, the moisture sensors may detect moisture in the chamber and may provide a signal to the controller or portable vacuum source to activate the portable suction device.

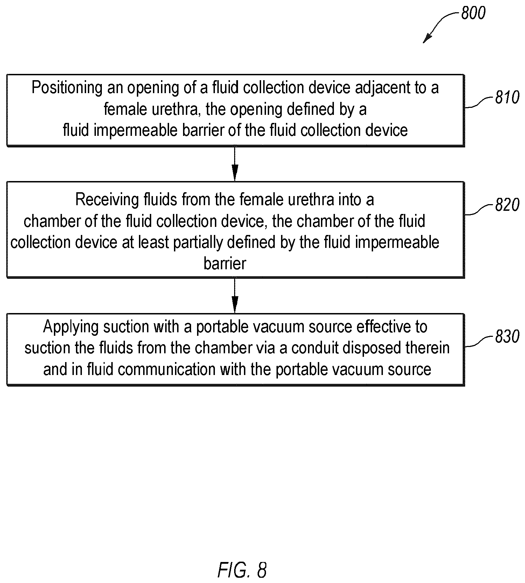

FIG. 8 is a flow diagram of a method 800 to use any of the fluid collection devices and/or fluid collection systems disclosed herein, according to an example. The method 800 can include act 810, which recites "positioning an opening of a fluid collection device adjacent to a female urethra or around a male urethra, the opening defined by a fluid impermeable barrier of the fluid collection device." Act 810 may be followed by act 820, which recites "receiving fluid from the female urethra or the male urethra into a chamber of the fluid collection device, the chamber of the fluid collection device at least partially defined by the fluid impermeable barrier." Act 820 may be followed by act 830, which recites "applying suction with a portable vacuum source effective to suction the fluid from the chamber via a conduit disposed therein and in fluid communication with the portable vacuum source."

Acts 810, 820, 830 of the method 800 are for illustrative purposes. For example, the act 810, 820, 830 of the method 800 can be performed in different orders, split into multiple acts, modified, supplemented, or combined. In an example, one or more of the acts 810, 820, 830 of the method 800 can be omitted from the method 800. Any of the acts 810, 820, or 830 can include using any of the fluid collection devices or systems disclosed herein.

Act 810 recites "positioning an opening of a fluid collection device adjacent to a female urethra, the opening defined by a fluid impermeable barrier of the fluid collection device." In some examples, act 810 can include positioning the opening of a female fluid collection device such that the fluid permeable membrane of the female fluid collection device abuts or is positioned proximate to the female urethra. In some examples, positioning an opening of a fluid collection device adjacent to a female urethra may include positioning the opening over the female urethra, such as positioning a longitudinally extending opening of the fluid collection device over the female urethra.

Act 820 recites "receiving fluid from the female urethra into a chamber of the fluid collection device, the chamber of the fluid collection device at least partially defined by the fluid impermeable barrier." For example, act 820 can include wicking the fluid(s) away from the opening using a fluid permeable membrane and a fluid permeable support. Act 820 can include flowing the fluid towards a portion of the chamber that is in fluid communication with an inlet of a conduit in fluid communication a vacuum source. For instance, act 820 can include flowing the fluid(s) to a substantially unoccupied portion of the chamber (e.g., a reservoir), to a gravimetrically low point of the chamber, etc. In some examples, receiving fluid(s) from the female urethra into a chamber of the fluid collection device may include wicking the fluid (e.g., urine) into the chamber via the fluid permeable membrane and fluid permeable support of the fluid collection device. For example, wicking the fluid into the chamber via the fluid permeable membrane and fluid permeable support may include wicking urine into a reservoir in the fluid collection device.

Act 830 recites, "applying suction with a portable vacuum source effective to suction the fluid(s) from the chamber via a conduit disposed therein and in fluid communication with the portable vacuum source." In some examples, applying suction with a portable vacuum source effective to suction the fluid(s) from the chamber via a conduit disposed therein and in fluid communication with the portable vacuum source can include using any of the portable vacuum sources disclosed herein. In an example, act 830 can include activating the portable vacuum source (e.g., portable suction device) in fluid communication with the inlet of the conduit in the fluid collection device. In some examples, activating the portable vacuum source in fluid communication with the inlet of the conduit in the fluid collection device can include supplying power to the portable vacuum source by one or more of flipping an on/off switch, pressing a button, plugging the portable vacuum source into a power outlet, putting batteries into the portable vacuum source, etc. In some examples, the portable vacuum source may include a hand operated vacuum pump and applying suction with a portable vacuum source may include manually operating the hand operated vacuum pump effective to suction the fluid(s) from the chamber via the conduit disposed therein that is in fluid communication with the portable vacuum source.

In some examples, applying suction with a portable vacuum source effective to suction the fluid(s) from the chamber via a conduit disposed therein and in fluid communication with the portable vacuum source can be effective to remove at least some fluid (e.g., urine) from the chamber (e.g., interior region) of the fluid collection device. In some examples, applying suction with a portable vacuum source effective to suction the fluid(s) from the chamber via a conduit disposed therein and in fluid communication with the portable vacuum source can be effective to transfer at least some of the fluid from the chamber of the fluid collection device to a fluid storage container (e.g., a bottle or bag). In some examples, applying suction with a portable vacuum source effective to suction the fluid(s) from the chamber may include removing fluid from one or more of a reservoir, fluid permeable support, or fluid permeable membrane of the fluid collection device.

The portable vacuum source (e.g., suction device) is disposed on or within the fluid collection device and applying suction with the portable vacuum source may include activating the portable vacuum source. Applying suction with the portable vacuum source may include activating the portable vacuum source.

In some examples, applying suction with a portable vacuum source effective to suction the fluid(s) from the chamber via a conduit disposed therein and in fluid communication with the portable vacuum source may include detecting moisture in the chamber (e.g., via one or more moisture sensors) and responsive thereto, activating the portable vacuum source to provide suction in the chamber. The control of the portable vacuum source responsive to the signals indicating that moisture or a level thereof is present in the chamber can be automatic, such as via a controller (e.g., computer programmed to perform the operation), or may merely provide an indication that a level of moisture is present that may necessitate removal of fluid from the chamber of the fluid collection device. In the latter case, a user may receive the indication (e.g., from the controller) and activate the portable vacuum pump manually.

In an example, the method 800 can include collecting the fluid(s) that are removed from the fluid collection device, such as into a fluid storage container that is spaced from the fluid collection device and in fluid communication with the conduit. The fluid storage container can include any of the fluid storage containers disclosed herein.

## Claims

1. A portable fluid collection system to be worn by a female user while working as a pilot or driver or in a hazardous area, and comprising:
a fluid storage container configured to hold a fluid;
a fluid collection device (300) in fluid communication with the fluid storage container, the fluid collection device having a longitudinal axis, a first end region and a second end region opposite the first end region and being shaped and sized to be positioned adjacent to a female urethra and including:
a fluid impermeable barrier (102) at least partially defining a chamber (104) with an interior region and a reservoir, the fluid impermeable barrier also defining an opening (106) extending therethrough, the opening configured to be positioned adjacent to a female urethra;
a conduit (108) including an inlet (110) and an outlet (112), the inlet being positioned within the fluid collection device at or near a gravimetrically low point of the chamber within the device when the device is being worn by a user and the outlet is configured to be in fluid communication with the fluid storage container whereby fluid collected in the fluid collection device may be removed from the interior region of the chamber via the conduit; and
a portable vacuum source (330) sized and shaped to fit on the outside of or within the fluid impermeable barrier of the fluid collection device, and in fluid communication with one or more of the fluid storage container or the fluid collection device, the portable vacuum source configured to draw fluid from the fluid collection device;
and wherein:
i) the fluid collection device and the fluid impermeable barrier defines a cylindrical shape with the opening (106) therein having a major axis and being longitudinally extending from a first location near the user's anus to a second location at or near the clitoris the opening exhibiting an elongated shape;
ii) the opening (106) with its major axis extending parallel to the longitudinal axis of the cylindrical shape of the fluid impermeable barrier and fluid collection device
iii) the conduit is at least partially disposed in the chamber and extends into the fluid impermeable barrier from the first end region proximate to the outlet to the second end region to a point proximate to the reservoir.

2. The fluid collection system of claim 1, further comprising:
a fluid permeable membrane (118) disposed within the chamber and extending across the longitudinally extending opening; and
a fluid permeable support (120) disposed within the chamber and positioned to support the fluid permeable membrane.

3. The fluid collection system of claim 2, wherein:
the fluid impermeable barrier and one or more of the fluid permeable membrane or fluid permeable support define the reservoir (122) therebetween; and
the inlet (110) is disposed in the reservoir.

4. The fluid collection system of any one of the preceding claims, wherein the portable vacuum source is disposed within the fluid impermeable barrier.

5. The fluid collection system of any one of claims 1 to 3, wherein the portable vacuum source is located on the fluid impermeable barrier.

6. System as claimed in any one of the preceding claims, wherein the opening exhibits a width that is greater than 50% of the circumference of the fluid collection device.

7. System as claimed in any one of the preceding claims and equipped with adhesive to attach the fluid impermeable barrier (102) to the user.

8. System as claimed in claim 7, wherein the adhesive is a hydrogel layer.

9. System as claimed in claim 2 or any one of claims 3 to 8 as dependent on claim 2, wherein the fluid permeable membrane (118) is a wicking material.

10. System as claimed in claim 2 or any one of claims 3 to 9 as dependent on claim 2, wherein the fluid permeable support (120) includes a material that can wick the fluid.

11. System as claimed in any one of the preceding claims and including one or more vacuum relief holes in the fluid impermeable barrier (102) to control an amount of suction or vacuum in the chamber (104).

## Patentansprüche

1. Tragbares Fluidsammelsystem, das von einem weiblichen Benutzer während des Arbeitens als eine Pilotin oder Fahrerin oder in einem Gefahrenbereich getragen werden soll, und umfassend:
einen Fluidspeicherbehälter, ausgebildet zum Halten eines Fluids;
eine Fluidsammelvorrichtung (300) in Fluidkommunikation mit dem Fluidspeicherbehälter, wobei die Fluidsammelvorrichtung eine longitudinale Achse, einen ersten Endbereich und einen zweiten Endbereich gegenüber dem ersten Endbereich aufweist, und gestaltet und bemessen ist, um angrenzend an eine weibliche Harnröhre positioniert zu werden, und einschließend:
eine fluidundurchlässige Barriere (102), die mindestens teilweise eine Kammer (104) mit einem Innenbereich und einem Reservoir definiert, wobei die fluidundurchlässige Barriere auch eine Öffnung (106) definiert, die sich dadurch erstreckt, wobei die Öffnung ausgebildet ist, um angrenzend an eine weibliche Harnröhre positioniert zu werden;
eine Leitung (108), die einen Einlass (110) und einen Auslass (112) einschließt, wobei der Einlass innerhalb der Fluidsammelvorrichtung an oder nahe einem gravimetrisch tiefsten Punkt der Kammer innerhalb der Vorrichtung positioniert ist, wenn die Vorrichtung von einer Benutzerin getragen wird, und der Auslass ausgebildet ist, um in Fluidkommunikation mit dem Fluidspeicherbehälter zu sein, wodurch in der Fluidsammelvorrichtung gesammeltes Fluid über die Leitung aus dem Innenbereich der Kammer entfernt werden kann; und
eine tragbare Vakuumquelle (330), die bemessen und gestaltet ist, um an der Außenseite oder innerhalb der fluidundurchlässigen Barriere der Fluidsammelvorrichtung zu passen, und in Fluidkommunikation mit einem oder mehreren aus dem Fluidspeicherbehälter oder der Fluidsammelvorrichtung, wobei die tragbare Vakuumquelle ausgebildet ist, um Flüssigkeit aus der Fluidsammelvorrichtung zu ziehen;
und wobei:
i) die Fluidsammelvorrichtung und die fluidundurchlässige Barriere eine zylindrische Gestalt definieren, wobei die Öffnung (106) darin eine Hauptachse aufweist und sich in longitudinaler Richtung von einem ersten Standort in der Nähe des Anus der Benutzerin zu einem zweiten Standort an oder in der Nähe der Klitoris erstreckt, wobei die Öffnung eine längliche Gestalt aufweist;
ii) die Öffnung (106) mit ihrer Hauptachse parallel zur longitudinalen Achse der zylindrischen Gestalt der fluidundurchlässigen Barriere und der Fluidsammelvorrichtung verläuft
iii) die Leitung mindestens teilweise in der Kammer angeordnet ist und sich in die fluidundurchlässige Barriere vom ersten Endbereich in der Nähe des Auslasses bis zum zweiten Endbereich zu einem Punkt in der Nähe des Reservoirs hinein erstreckt.

2. Fluidsammelsystem nach Anspruch 1, weiter umfassend:
eine fluiddurchlässige Membran (118), die innerhalb der Kammer angeordnet ist und sich über die sich longitudinal erstreckende Öffnung erstreckt; und
einen fluiddurchlässigen Träger (120), der innerhalb der Kammer angeordnet und positioniert ist, um die fluiddurchlässige Membran zu stützen.

3. Fluidsammelsystem nach Anspruch 2, wobei:
die fluidundurchlässige Barriere und eines oder mehrere aus der fluiddurchlässigen Membran oder dem fluiddurchlässigen Träger das Reservoir (122) dazwischen definieren; und der Einlass (110) im Reservoir angeordnet ist.

4. Fluidsammelsystem nach einem der vorstehenden Ansprüche, wobei die tragbare Vakuumquelle innerhalb der fluidundurchlässigen Barriere angeordnet ist.

5. Fluidsammelsystem nach einem der Ansprüche 1 bis 3, wobei die tragbare Vakuumquelle auf der fluidundurchlässigen Barriere lokalisiert ist.

6. System nach einem der vorstehenden Ansprüche, wobei die Öffnung eine Breite aufweist, die größer ist als 50 % des Umfangs der Fluidsammelvorrichtung.

7. System nach einem der vorstehenden Ansprüche und ausgestattet mit Klebstoff, um die fluidundurchlässige Barriere (102) an der Benutzerin anzubringen.

8. System nach Anspruch 7, wobei der Klebstoff eine Hydrogelschicht ist.

9. System nach Anspruch 2 oder einem der Ansprüche 3 bis 8, wie abhängig von Anspruch 2, wobei die fluiddurchlässige Membran (118) ein kapillares Material ist.

10. System nach Anspruch 2 oder einem der Ansprüche 3 bis 9, wie abhängig von Anspruch 2, wobei der fluiddurchlässige Träger (120) ein Material einschließt, das das Fluid kapillar leiten kann.

11. System nach einem der vorstehenden Ansprüche, und einschließend ein oder mehrere Vakuumentlastungslöcher in der fluidundurchlässigen Barriere (102), um die Stärke der Saugkraft oder des Vakuums in der Kammer (104) zu steuern.

## Revendications

1. Système portable de collecte de fluide destiné à être porté par une utilisatrice pendant qu'elle travaille en tant que pilote ou conductrice ou dans une zone dangereuse, et comprenant :
un récipient de stockage de fluide configuré pour contenir un fluide ;
un dispositif (300) de collecte de fluide en communication fluidique avec le récipient de stockage de fluide, le dispositif de collecte de fluide présentant un axe longitudinal, une première région d'extrémité et une deuxième région d'extrémité opposée à la première région d'extrémité et étant formé et dimensionné pour être positionné adjacent à un urètre féminin et incluant :
une barrière (102) imperméable aux fluides définissant au moins partiellement une chambre (104) avec une région intérieure et un réservoir, la barrière imperméable aux fluides définissant également une ouverture (106) s'étendant à travers celle-ci, l'ouverture étant configurée pour être positionnée adjacente à un urètre féminin ;
un conduit (108) incluant une entrée (110) et une sortie (112), l'entrée étant positionnée à l'intérieur du dispositif de collecte de fluide au niveau de ou à proximité d'un point gravimétriquement bas de la chambre à l'intérieur du dispositif lorsque le dispositif est porté par une utilisatrice et la sortie est configurée pour être en communication fluidique avec le récipient de stockage de fluide de sorte que le fluide collecté dans le dispositif de collecte de fluide puisse être retiré de la région intérieure de la chambre via le conduit ; et
une source de vide portable (330) dimensionnée et formée pour s'adapter sur l'extérieur de ou à l'intérieur de la barrière imperméable aux fluides du dispositif de collecte de fluide, et en communication fluidique avec un ou plusieurs parmi le récipient de stockage de fluide ou le dispositif de collecte de fluide, la source de vide portable étant configurée pour aspirer du fluide depuis le dispositif de collecte de fluide ;
et dans lequel :
i) le dispositif de collecte de fluide et la barrière imperméable aux fluides définissent une forme cylindrique avec l'ouverture (106) à l'intérieur de celle-ci présentant un grand axe et s'étendant longitudinalement d'un premier emplacement près de l'anus de l'utilisatrice à un deuxième emplacement au niveau de ou à proximité du clitoris, l'ouverture présentant une forme allongée ;
ii) l'ouverture (106) avec son grand axe s'étendant parallèlement à l'axe longitudinal de la forme cylindrique de la barrière imperméable aux fluides et du dispositif de collecte de fluide
iii) le conduit est au moins partiellement disposé dans la chambre et s'étend dans la barrière imperméable aux fluides depuis la première région d'extrémité à proximité de la sortie jusqu'à la deuxième région d'extrémité jusqu'à un point à proximité du réservoir.

2. Système de collecte de fluide selon la revendication 1, comprenant en outre :
une membrane (118) perméable aux fluides disposée à l'intérieur de la chambre et s'étendant à travers l'ouverture s'étendant longitudinalement ; et
un support (120) perméable aux fluides disposé à l'intérieur de la chambre et positionné pour supporter la membrane perméable aux fluides.

3. Système de collecte de fluide selon la revendication 2, dans lequel :
la barrière imperméable aux fluides et un ou plusieurs parmi la membrane perméable aux fluides ou le support perméable aux fluides définissent le réservoir (122) entre eux ; et l'entrée (110) est disposée dans le réservoir.

4. Système de collecte de fluide selon l'une quelconque des revendications précédentes, dans lequel la source de vide portable est disposée à l'intérieur de la barrière imperméable aux fluides.

5. Système de collecte de fluide selon l'une quelconque des revendications 1 à 3, dans lequel la source de vide portable est située sur la barrière imperméable aux fluides.

6. Système selon l'une quelconque des revendications précédentes, dans lequel l'ouverture présente une largeur qui est supérieure à 50 % de la circonférence du dispositif de collecte de fluide.

7. Système selon l'une quelconque des revendications précédentes et pourvu d'un adhésif pour fixer la barrière (102) imperméable aux fluides à l'utilisatrice.

8. Système selon la revendication 7, dans lequel l'adhésif est une couche d'hydrogel.

9. Système selon la revendication 2 ou l'une quelconque des revendications 3 à 8 lorsqu'elles dépendent de la revendication 2, dans lequel la membrane (118) perméable aux fluides est un matériau à effet de mèche.

10. Système selon la revendication 2 ou l'une quelconque des revendications 3 à 9 lorsqu'elles dépendent de la revendication 2, dans lequel le support (120) perméable aux fluides inclut un matériau capable d'acheminer le fluide par capillarité.

11. Système selon l'une quelconque des revendications précédentes et incluant un ou plusieurs orifices de mise à l'air dans la barrière (102) imperméable aux fluides pour commander une quantité d'aspiration ou de vide dans la chambre (104).
